# FASCICULE DE BREVET EUROPEEN

(11) **EP 0 566 438 B1**
(45) Date de publication et mention de la délivrance du brevet: **04.11.1998**
(21) Numéro de dépôt: 93400821.0
(22) Date de dépôt: 30.03.1993
(51) Int. Cl.: C07H 13/06, A61K 47/26, A61K 7/00, A61K 7/16, A61K 9/133

(54) **Procédé de préparation de monoesters majoritairement en position 6' du D-maltose et leur utilisation dans les domaines cosmétique, bucco-dentaire, pharmaceutique et alimentaire**
Verfahren zur Herstellung von D-Maltose-Monoestern mit einem hohen Gehalt von 6-0'-Ester und ihre Verwendung in Kosmetika, Zahnpflegemitteln, Pharmazeutik und Lebensmitteln
Method for the preparation of D-maltose monoesters with a high 6-0'-ester content and their use in the cosmetic, dental-care, pharmaceuticals and food stuff fields

(30) Priorité: 30.03.1992 FR 9203812
(43) Date de publication de la demande: 20.10.1993
(73) Titulaire: L'OREAL, 75008 Paris (FR)
(72) Inventeur: Philippe, Michel, 91320 Wissous (FR)
(74) Mandataire: Stalla-Bourdillon, Bernard

(56) Documents cités:
- WO-A-91/01322
- CHEMICAL AND PHARMACEUTICAL BULLETIN. vol. 29, no. 2, 1981, TOKYO JP pages 505 - 513 NISHIKAWA Y. ET AL 'Chemical and Biochemical Studies on Carbohydate Esters. IX. Antitumor Effects of Selectively Fatty Acylated Products of Maltose'

## Description

La présente invention a pour objet un nouveau procédé de préparation de monoesters majoritairement en position 6' du D-maltose et leur utilisation dans différents domaines de l'industrie tels que le domaine cosmétique, bucco-dentaire, pharmaceutique et alimentaire.

Diverses synthèses d'esters de polyols, en particulier de disaccharides, ont été proposées mais aucune d'entre elles ne décrit la préparation de monoesters majoritairement en position 6' du D-maltose.

Parmi les procédés récents de préparation d'esters de polyols, on peut notamment citer le procédé décrit dans la demande de brevet WO 91/01322, consistant en une première étape à préparer, en milieu aqueux, un anhydride mixte carboxylique-carbonique de l'acide à esterifier et dans une deuxième étape, à faire réagir ledit anhydride mixte avec le polyol.

Cette méthode de synthèse en milieu aqueux ne permet cependant pas d'accéder aux monoesters en 6' du D-maltose.

On peut également citer, comme autre procédé de préparation d'esters de polyols, l'article de Nishikawa et al. (Chem. Pharm. Bull. 29(2), 1981, pp. 505-513) qui consiste à réaliser une réaction d'acylation du maltose à l'aide d'un chlorure d'acide approprié en présence de pyridine. Ce procédé conduit toutefois à un mélange isomérique de monoesters dont les isomères sont de façon prédominante en position 1, 6 et 6'.

Après diverses études, on a maintenant constaté de façon inattendue qu'il était possible d'obtenir majoritairement les monoesters en 6' du D-maltose par l'intermédiaire de la méthode à l'anhydride mixte carboxylique-carbonique mais en réalisant la synthèse en milieu solvant organique alors que selon la demande de brevet WO 91/01322, comme indiqué ci-dessus, celle-ci est réalisée en milieu aqueux, de préférence dans l'eau.

La présente invention a donc pour objet un procédé de préparation de monoesters majoritairement en position 6' du D-maltose, celui-ci consistant en un premier temps à former dans un solvant organique, un anhydride mixte de formule (I) : 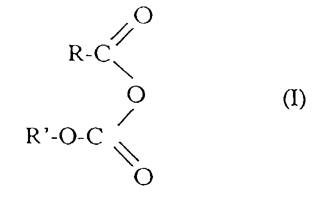 dans laquelle :
R représente un radical alkyle, linéaire ou ramifié, ou alcényle, ayant de 7 à 21 atomes de carbone ou R représente un mélange défini de tels radicaux alkyles ou alcényles, et R' représente un radical alkyle, linéaire ou ramifié, ayant de 2 à 10 atomes de carbone, par réaction, en présence d'une base, d'un acide R-COOH, R ayant la même signification que ci-dessus, et d'un chloroformiate d'alkyle ClCOOR', R' ayant la même signification que ci-dessus, et dans un deuxième temps, à faire réagir ledit anhydride mixte de formule (I) en solution dans ledit solvant organique avec du D-maltose.

Comme solvant organique réactionnel, on peut utiliser selon l'invention le tétrahydrofuranne, le N,N-diméthylformamide ou la N-méthyl-pyrrolidone.

La base servant à l'activation de l'acide est de préférence une base organique choisie parmi la triéthylamine, la pyridine, la 4-diméthylaminopyridine, la tributylamine ou encore la N-méthylmorpholine.

La deuxième étape, ou étape d'estérification, peut être éventuellement réalisée après essorage des sels formés lors de la première étape et le D-maltose est de préférence en solution dans la pyridine, ou éventuellement dans le diméthylformamide, la N-méthylpyrrolidone ou le diméthylacétamide.

On utilise de préférence selon l'invention au moins trois équivalents de D-maltose par rapport à l'acide mis à réagir dans la première étape.

Selon l'invention, le chloroformiate d'alkyle est de préférence choisi parmi le chloroformiate d'éthyle et le chloroformiate d'isopropyle.

Après la fin de la réaction, les solvants sont évaporés et le produit résultant peut être chromatographié sur colonne de gel de silice en utilisant de préférence un mélange de solvants à base de chlorure de méthylène et de méthanol.

La température de réaction du procédé est généralement comprise entre -25 et +40°C et le temps de réaction entre 3 et 15 heures.

L'acide R-COOH est de préférence choisi parmi l'acide octanoïque, l'acide dodécanoïque, l'acide tétradécanoïque, l'acide hexadécanoïque et l'acide oléïque.

Les monoesters majoritairement en position 6' du D-maltose obtenu par le procédé selon l'invention tel que décrit ci-dessus peuvent être représentés par la formule générale suivante : 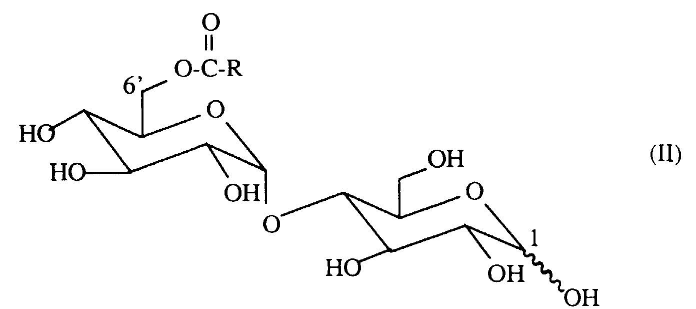 dans laquelle :
R représente un radical alkyle, linéaire ou ramifié, ou alcényle, ayant de 7 à 21 atomes de carbone ou R représente un mélange défini de tels radicaux alkyles ou alcényles, le monoester en position 6' représentant environ au moins 70 % en poids, le reste étant essentiellement constitué par le monoester en position 1.

Parmi les monoesters majoritairement en position 6' du D-maltose de formule (II) selon l'invention, on peut notamment mentionner les suivants :
6'-O-octanoyl-D-maltose,
6'-O-hexadécanoyl-D-maltose,
6'-O-oléoyl-D-maltose,
6'-O-dodécanoyl-D-maltose, et
6'-O-tétradécanoyl-D-maltose.

La détermination des structures des produits obtenus a été réalisée par RMN¹³C et ¹H (250 MH_{Z} dans le diméthylsulfoxyde (DMSO) deutérié.

Le procédé de l'invention par rapport aux procédés connus permet de conduire à des monoesters de D-maltose parfaitement définis quant à leur structure et à leur composition, ce qui est un avantage majeur par rapport aux esters de maltose connus qui sont le plus souvent des mélanges de mono et polyesters.

Ceci est tout à fait primordial pour la valorisation et l'utilisation de ces composés notamment dans les domaines cosmétique, pharmaceutique, bucco-dentaire et alimentaire.

En effet, grâce à une bonne caractérisation des monoesters, on peut ainsi mieux maîtriser l'efficacité et l'innocuité des compositions les contenant.

Les monoesters du D-maltose de formule (II) telle que définie ci-dessus présentent, par rapport aux esters connus, de très intéressantes propriétés et trouvent notamment une application dans les domaines cosmétique, pharmaceutique et alimentaire.

Parmi ces propriétés, on doit tout particulièrement mentionner celle permettant d'abaisser la tension de surface qui n'est pas modifiée même en présence de solutions salines (NaCl 0,1M et CaCl₂ 0,033M). Par ailleurs, les monoesters du D-maltose de formule (II), notamment lorsque R représente un radical alkyle ayant de 13 à 21 atomes de carbone, peuvent former en association avec le cholestérol et le dicétylphosphate de sodium par exemple dans des proportions respectives de 47,5/47,5/5 ou 62,5/32,5/5, des vésicules. A l'état hydraté, ces associations permettent, en effet, d'obtenir des phases lamellaires Lα dès la température ambiante, ces phases lamellaires étant dispersibles sous forme de vésicules.

Les monoesters du D-maltose de formule (II) présentent également un faible pouvoir hémolytique notamment par rapport aux dérivés correspondants de glucose. En outre, ils présentent une cytotoxicité très nettement inférieure à celle des dérivés analogues de glucose, notamment à ceux comportant une chaîne oléique.

Il convient enfin de souligner que les monoesters du D-maltose de formule (II) sont entièrement biodégradables et moins écotoxiques que les dérivés du glucose du commerce, tout en présentant une bonne stabilité chimique.

La présente invention a donc également pour objet l'utilisation des monoesters du D-maltose de formule (II) en tant qu'agents détergents, agents moussants, agents émulsionnants, agents contribuant à l'hydratation de la peau et substances aptes à former des vésicules.

En cosmétique, les monoesters du D-maltose trouvent tout particulièrement une utilisation comme détergents doux pour la peau ou les cheveux.

La présente invention a donc également pour objet une composition cosmétique, bucco-dentaire, pharmaceutique ou alimentaire contenant dans un véhicule approprié, au moins un monoester du D-maltose de formule (II) tel que défini ci-dessus.

Dans les compositions selon l'invention, le monoester du D-maltose est généralement présent à une concentration comprise entre 0,01 et 30 %, mais de préférence entre 0,5 et 15 % en poids par rapport au poids total de la composition.

Les compositions selon l'invention peuvent se présenter sous différentes formes, notamment sous forme de lotions, moussantes ou non moussantes, d'émulsions de consistance liquide ou semi-liquide telles que des laits obtenus par dispersion d'une phase grasse dans une phase aqueuse ou inversement, de suspensions ou d'émulsions de consistance molle du type crèmes ou pommades, de gels ou encore de préparations solides telles que des sticks, des pains de nettoyage, des tampons imprégnés ou encore sous forme de masques hydratants.

Comme véhicule des compositions selon l'invention, on peut utiliser de l'eau, des solvants organiques compatibles avec une application topique tels que l'acétone, l'alcool isopropylique, l'alcool éthylique, les triglycérides d'acides gras en C₆-C₂₄, les éthers de glycol tels que les éthers d'alkyle inférieur de mono ou dialkylène glycol, dont le radical alkylène a de 2 à 4 atomes de carbone.

On peut également utiliser comme solvant des esters de polyalkylèneglycol et d'acide à chaîne courte en C₁-C₄ ou encore des silicones volatiles.

Les compositions selon l'invention peuvent également contenir des corps gras tels que des huiles naturelles ou synthétiques.

Les compositions selon l'invention peuvent également contenir des agents épaississants ou gélifiants tels que la cellulose ou des dérivés de cellulose. Les agents épaississants peuvent être également des polymères acryliques, des alginates, des gommes telles que la gomme de xanthane, de guar, de caroube ou la gomme arabique ou encore des polyéthylène glycols, des bentonites et des montmorillonites.

Les compositions selon l'invention peuvent également contenir des matières actives comme des agents hydratants ainsi que des adjuvants tels que des agents antioxydants, des agents conservateurs, des parfums, des colorants.

Les compositions selon l'invention peuvent également se présenter sous forme de solutions ou de dispersions contenant les monoesters du D-maltose sous forme vésiculaire, les vésicules pouvant alors servir d'agents d'encapsulation pour des ingrédients actifs lipophiles et/ou hydrophiles.

### PROCEDE GENERAL DE PREPARATION DES 6'-O-ALCANOYL-D-MALTOSES

L'acide carboxylique R-COOH est mis en solution dans un solvant organique tel que le tétrahydrofuranne à 25 % (P/V). A cette solution on ajoute 1,05 équivalents de triéthylamine et le milieu est agité à température ambiante pendant 1 heure. La solution du sel de triéthylamine obtenue est alors ajoutée à une solution de chloroformiate d'alkyle ClCOOR'de préférence le chloroformiate d'isopropyle (1 eq.) dans le tétrahydrofuranne (10 % P/V) refroidie à -20°C avec un bain alcool/carboglace. Après addition, le milieu est laissé sous agitation au minimum 3 heures.

Le D-maltose (≃ 3 eq.) est parallèlement mis en solution dans un solvant basique tel que la pyridine anhydre (7 ml/g) et le milieu précédent, éventuellement filtré, est additionné à la solution de D-maltose à température ambiante pendant au moins 3 heures.

Après évaporation des solvants, le produit est chromatographié sur colonne de gel de silice en utilisant un mélange de solvants à base de chlorure de méthylène et de méthanol.

La purification par chromatographie peut éventuellement être substituée par une extraction liquide/liquide.

Selon ce procédé général, on a préparé les 6'-O-alcanoyl-D-maltoses suivants :

### EXEMPLE 1: 6'-O-octanoyl-D-maltose

Rendement = 35 %
F = 98°C

| Analyse élémentaire : C₂₀H₃₆O₁₂, 0,5H₂O ; M = 477,5 | | |
|---|---|---|
| | C % | H % |
| Calc. | 50,30 | 7,81 |
| Tr. | 50,07 | 7,62 |

La RMN du ¹³C (DMSO D₆) est conforme à la structure attendue.

### EXEMPLE 2 : 6'-O-hexadécanoyl-D-maltose

Rendement = 40 %
F = 95°C

| Analyse élémentaire : C₂₈H₅₂O₁₂, 0,5H₂O ; M = 589,7 | | |
|---|---|---|
| | C % | H % |
| Calc. | 57,03 | 9,05 |
| Tr. | 56,62 | 8,64 |

La RMN du ¹³C (DMSO D₆) est conforme à la structure attendue.

### EXEMPLE 3 : 6'-O-oléoyl-D-maltose

Rendement = 50 %
F = 82°C

| Analyse élémentaire : C₃₀H₅₄O₁₂, 1,5H₂O ; M = 633,8 | | |
|---|---|---|
| | C % | H % |
| Calc. | 56,85 | 9,06 |
| Tr. | 56,55 | 8,78 |

La RMN du ¹³C (DMSO D₆) est conforme à la structure attendue.

### EXEMPLE 4 : 6'-O-dodécanoyl-D-maltose

Rendement = 40 %
F = 93°C

| Analyse élémentaire : C₂₄H₄₄O₁₂, O,5H₂O ; M = 533,6 | | |
|---|---|---|
| | C % | H % |
| Calc. | 54,02 | 8,50 |
| Tr. | 54,06 | 8,39 |

Spectre RMN¹³C et ¹H (250 MHz dans le DMSO D6+D₂O)

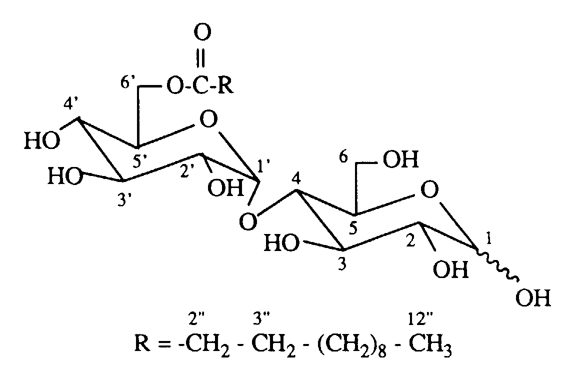

| Pics¹H (ppm) | | | Pics¹³C (ppm) | |
|---|---|---|---|---|
| O,85 | triplet | H12'' | 13,98 | C12'' |
| 1,23 | multiplet | (CH₂)₈ | 22,12 | C11'' |
| 1,50 | multiplet | H3'' | 24,14 à 24,51 | C3'' |
| 2,30 | triplet | H2'' | 28,40 à 29,03 | (CH₂)₆ |
| 2,94 à 3,62 | multiplets | H2 à H6 - H2' à H5' | 31,31 | C10'' |
| 4,02 | multiplet | H6' | 39,34-33,38 | C2'' |
| 4,26 | multiplet | H6' | 60,11 à 60,77 | C6 et C6' non substitués |
| 4,38 | H1β | | 63,55 | C6' substitué |
| 4,54 | OH | | 70,00 à 81,12 | C2' à C5' et C2 à C5 |
| 4,90 à 5,00 | multiplet | H1'-H1α | 92,08 | C1α |
| 5,53 | OH | | 96,79 | C1β |
| | | | 100,81 | C1' |
| | | | 172,95 | C1'' |

| | | | Pic supplémentaire | |
|---|---|---|---|---|
| | | | 93,94 | C1β maltose substitué en 1 (23 %) |

### EXEMPLE 5 : 6'-O-tétradécanoyl-D-maltose

Rendement 55 %
F = 110°C

| Analyse élémentaire : C₂₆H₄₈O₁₂, 0,5H₂O ; M = 561,7 | | |
|---|---|---|
| | C % | H % |
| Calc. | 55,6 | 8,79 |
| Tr. | 55,44 | 8,84 |

La RMN du ¹³C (DMSO D₆) est conforme à la structure attendue.

### EXEMPLES DE COMPOSITIONS COSMETIQUES

### EXEMPLE 1 : Préparation vésiculaire sous forme de crème épaisse

On prépare tout d'abord une phase lipidique en procédant au mélange des ingrédients suivants :
- 6'-O-hexadécanoyl-D-maltose obtenu selon l'Exemple 2 47,53 g
- Cholestérol 47,53 g
- Dicétylphosphate de sodium 4,94 g

Cette phase lipidique dans une proportion de 3 à 10 % en poids est ensuite utilisée pour la préparation de la composition vésiculaire selon les méthodes classiques, le reste de la composition étant constitué par de l'eau éventuellement additionnée d'agents de conservation et/ou antioxydants et éventuellement d'huile.

### EXEMPLE 2 : Préparation vésiculaire sous forme de crème fluide

On prépare tout d'abord une phase lipidique en procédant au mélange des ingrédients suivants :
- 6'-O-tétradécanoyl-D-maltose obtenu selon l'Exemple 5 62,53 g
- Cholestérol 32,53 g
- Dicétylphosphate de sodium 4,94 g

Cette phase lipidique dans une proportion de 3 à 10 % en poids est ensuite utilisée pour la préparation de la composition vésiculaire selon les méthodes classiques, le reste de la composition étant constitué par de l'eau éventuellement additionnée d'agents de conservation et/ou antioxydants et éventuellement d'huile.

### EXEMPLE 3 : Préparation moussante

- 6'-O-dodécanoyl-D-maltose obtenu selon l'exemple 4 5 g
- Laurylsulfate de triéthanolamine à 40 % dans l'eau 40 g
- Cocoyl bétaïne 4 g
- Diéthanolamide d'acides de coprah 3 g
- Conservateurs 0,2 g
- Eau qsp 100 g

### EXEMPLE 4 : Préparation sous forme d'émulsion

- 6'-O-hexadécanoyl-D-maltose obtenu selon l'Exemple 2 5 g
- Huile de vaseline 20 g
- Hydroxyéthylcellulose 0,5 g
- Imidazolidinyl urée 0,2 g
- Eau qsp 100 g

## Revendications

1. Procédé de préparation de monoesters majoritairement en position 6' du D-maltose, caractérisé par le fait qu'il consiste en un premier temps à former dans un solvant organique, un anhydride mixte de formule (I) : 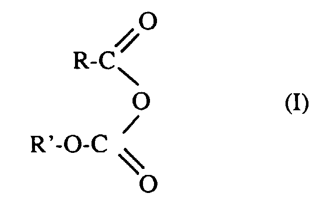 dans laquelle :
R représente un radical alkyle, linéaire ou ramifié, ou alcényle, ayant de 7 à 21 atomes de carbone ou R représente un mélange défini de tels radicaux alkyles ou alcényles,
et R' représente un radical alkyle, linéaire ou ramifié ayant de 2 à 10 atomes de carbone, par réaction, en présence d'une base, d'un acide R-COOH, R ayant la même signification que ci-dessus, et d'un chloroformiate d'alkyle ClCOOR', R' ayant la même signification que ci-dessus et dans un deuxième temps à faire réagir ledit anhydride mixte de formule (I) en solution dans ledit solvant organique avec du D-maltose.

2. Procédé selon la revendication 1, caractérisé par le fait que le solvant organique est choisi parmi le tétrahydrofuranne, le N,N-diméthylformamide ou la N-méthyl-pyrrolidone.

3. Procédé selon la revendication 1, caractérisé par le fait que la base est choisie parmi la triéthylamine, la pyridine, la 4-diméthylaminopyridine, la tributylamine ou la N-méthylmorpholine.

4. Procédé selon la revendication 1, caractérisé par le fait que le D-maltose est mis à réagir en solution dans de la pyridine, du diméthylformamide, de la N-méthylpyrrolidone ou du diméthylacétamide.

5. Procédé selon l'une quelconque des revendications précédentes, caractérisé par le fait que l'on fait réagir environ trois équivalents de D-maltose par rapport à l'acide.

6. Procédé selon l'une quelconque des revendications précédentes, caractérisé par le fait qu'après la fin de la réaction et évaporation des solvants, on procède à une chromatographie sur colonne de gel de silice.

7. Procédé selon l'une quelconque des revendications 1 à 5, caractérisé par le fait qu'après la fin de la réaction et évaporation éventuelle des solvants, on procède à une extraction liquide/liquide.

8. Monoesters majoritairement en position 6' du D-maltose obtenus par le procédé selon les revendications 1 à 7, caractérisés par le fait qu'ils répondent à la formule générale suivante : 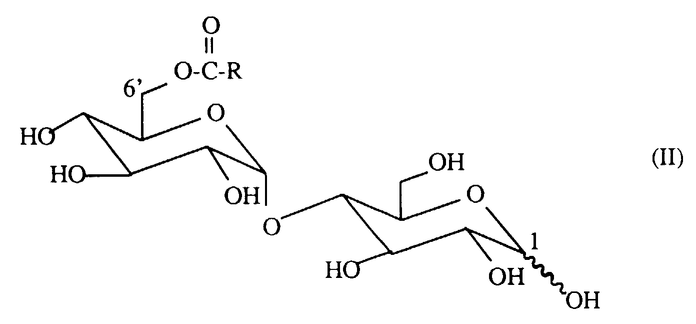 dans laquelle :
R représente un radical alkyle, linéaire ou ramifié, ou alcényle, ayant de 7 à 21 atomes de carbone ou R représente un mélange défini de tels radicaux alkyles ou alcényles, le monoester en position 6' représentant environ au moins 70 % en poids, le reste étant essentiellement constitué par le monoester en position 1.

9. Monoesters du D-maltose selon la revendication 8, caractérisés par le fait qu'ils sont choisis parmi :
6'-O-octanoyl-D-maltose,
6'-O-hexadécanoyl-D-maltose,
6'-O-oléoyl-D-maltose,
6'-O-dodécanoyl-D-maltose, et
6'-O-tétradécanoyl-D-maltose.

10. Composition cosmétique, bucco-dentaire, pharmaceutique ou alimentaire, caractérisée par le fait qu'elle contient dans un véhicule approprié au moins un monoester majoritairement en position 6' du D-maltose de formule (II) selon les revendications 8 et 9 ou obtenue selon l'une quelconque des revendications 1 à 7.

11. Composition selon la revendication 10, caractérisée par le fait que le monoester du D-maltose est présent à une concentration comprise entre 0,01 et 30 % et de préférence entre 0,5 et 15 % en poids par rapport au poids total de la composition.

## Patentansprüche

1. Verfahren zur Herstellung von mehrheitlich in 6'-Position veresterten Monoestern der D-Maltose, dadurch gekennzeichnet, daß das Verfahren aus einem ersten Schritt, bei dem in einem organischen Lösungsmittel ein gemischtes Anhydrid der Formel (I) gebildet wird: 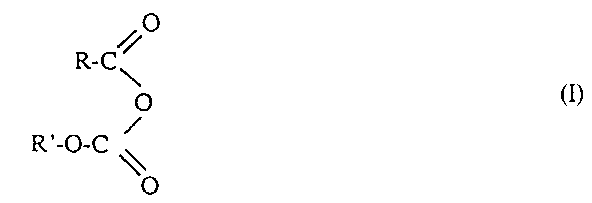 worin
R für einen linearen oder verzweigten Alkyl- oder Alkenylrest mit 7 bis 21 Kohlenstoffatomen steht oder R für eine definierte Mischung dieser Alkyl- oder Alkenylreste steht, und R' für einen linearen oder verzweigten Alkylrest mit 2 bis 10 Kohlenstoffatomen steht, durch Reaktion einer Säure R-COOH, worin R dieselbe Bedeutung wie oben aufweist, mit einem Alkylchlorformiat ClCOOR', worin R' dieselbe Bedeutung wie oben aufweist, in Gegenwart einer Base, und aus einem zweiten Schritt besteht, bei dem man das gemischte Anhydrid der Formel (I) in Lösung in dem organischen Lösungsmittel mit D-Maltose reagieren läßt.

2. Verfahren nach Anspruch 1, dadurch gekennzeichnet, daß das organische Lösungsmittel gewählt ist aus Tetrahydrofuran, N,N-Dimethylformamid oder N-Methylpyrrolidon.

3. Verfahren nach Anspruch 1, dadurch gekennzeichnet, daß die Base gewählt ist aus Triethylamin, Pyridin, 4-Dimethylaminopyridin, Tributylamin oder N-Methylmorpholin.

4. Verfahren nach Anspruch 1, dadurch gekennzeichnet, daß man die D-Maltose in Lösung in Pyridin, Dimethylformamid, N-Methylpyrrolidon oder Dimethylacetamid reagieren läßt.

5. Verfahren nach einem der vorangehenden Ansprüche, dadurch gekennzeichnet, daß man ungefähr drei Äquivalente D-Maltose, bezogen auf die Säure, umsetzt.

6. Verfahren nach einem der vorangehenden Ansprüche, dadurch gekennzeichnet, daß nach Beendigung der Reaktion und dem Verdampfen der Lösungsmittel eine Chromatographie an einer Silicagelsäule durchgeführt wird.

7. Verfahren nach einem der Ansprüche 1 bis 5, dadurch gekennzeichnet, daß nach Beendigung der Reaktion und dem eventuellen Verdampfen der Lösungsmittel eine Flüssig/Flüssig-Extraktion durchgeführt wird.

8. Mehrheitlich in 6-Position veresterte Monoester der D-Maltose, erhalten durch das Verfahren nach den Ansprüchen 1 bis 7, dadurch gekennzeichnet, daß sie wiedergegeben werden durch die folgende allgemeine Formel: 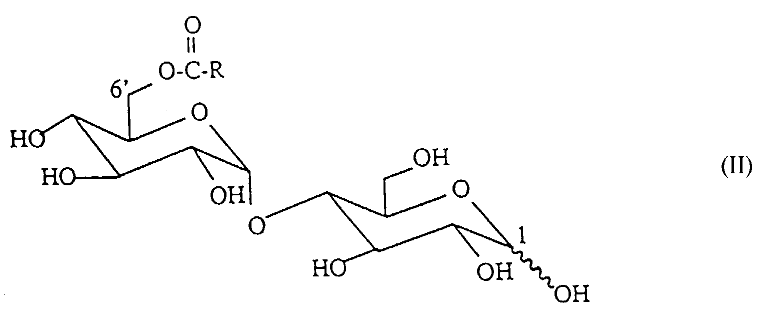 worin
R für einen linearen oder verzweigten Alkyl- oder Alkenylrest mit 7 bis 21 Kohlenstoffatomen steht oder R für eine definierte Mischung dieser Alkyl- oder Alkenylreste steht, wobei der in 6'-Position veresterte Monoester ungefähr wenigstens 70 Gew.-% ausmacht und der Rest im wesentlichen aus dem in Position 1 veresterten Monoester besteht.

9. D-Maltose-Monoester nach Anspruch 8, dadurch gekennzeichnet, daß sie gewählt sind aus
- 6'-O-Octanoyl-D-maltose;
- 6'-O-Hexadecanoyl-D-maltose;
- 6'-O-Oleoyl-D-maltose;
- 6'-O-Dodecanoyl-D-maltose; und
- 6'-O-Tetradecanoyl-D-maltose.

10. Kosmetische, oral-zahnärztliche, pharmazeutische oder Nahrungszubereitung, dadurch gekennzeichnet, daß sie in einem geeigneten Träger wenigstens einen mehrheitlich in 6'-Position veresterten Monoester der D-Maltose der Formel (II) nach den Ansprüchen 8 und 9 oder erhalten nach irgendeinem der Ansprüche 1 bis 7 enthält.

11. Zubereitung nach Anspruch 10, dadurch gekennzeichnet, daß der Monoester der D-Maltose in einer Konzentration zwischen 0,01 und 30 Gew.-%, vorzugsweise zwischen 0,5 und 15 Gew.-%, vorhanden ist, bezogen auf das Gesamtgewicht der Zubereitung.

## Claims

1. Method for the preparation of D-maltose monoesters predominantly in the 6' position, characterized in that it consists, in a first step, in forming, in an organic solvent, a mixed anhydride of formula (I) : 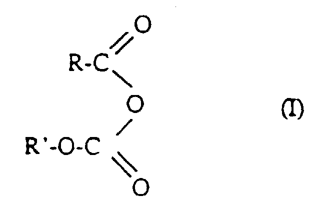 in which:
R represents a linear or branched alkyl radical or an alkenyl radical having from 7 to 21 carbon atoms or R represents a defined mixture of such alkyl or alkenyl radicals,
and R' represents a linear or branched alkyl radical having from 2 to 10 carbon atoms,
by reaction, in the presence of a base, of an acid R-COOH, R having the same meaning as above, and of an alkyl chloroformate ClCOOR', R' having the same meaning as above, and, in a second step, in reacting the said mixed anhydride of formula (I), in solution in the said organic solvent, with D-maltose.

2. Method according to Claim 1, characterized in that the organic solvent is chosen from tetrahydrofuran, N,N-dimethylformamide or N-methylpyrrolidone.

3. Method according to Claim 1, characterized in that the base is chosen from triethylamine, pyridine, 4-dimethylaminopyridine, tributylamine or N-methylmorpholine.

4. Method according to Claim 1, characterized in that D-maltose is reacted in solution in pyridine, dimethylformamide, N-methylpyrrolidone or dimethylacetamide.

5. Method according to any one of the preceding claims, characterized in that approximately three equivalents of D-maltose are reacted with respect to the acid.

6. Method according to any one of the preceding claims, characterized in that, after the end of the reaction and evaporation of the solvents, a chromatography is carried out on a silica gel column.

7. Method according to any one of Claims 1 to 5, characterized in that, after the end of the reaction and optional evaporation of the solvents, a liquid/liquid extraction is carried out.

8. D-Maltose monoesters predominantly in the 6' position obtained by the method according to Claims 1 to 7, characterized in that they correspond to the following general formula: 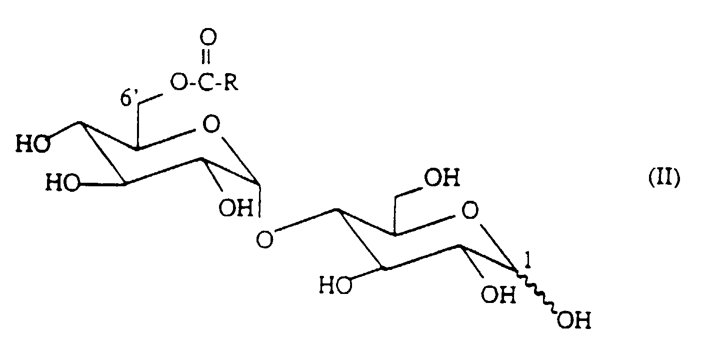 in which:
R represents a linear or branched alkyl radical or an alkenyl radical having from 7 to 21 carbon atoms or R represents a defined mixture of such alkyl or alkenyl radicals, the monoester in the 6' position representing approximately at least 70% by weight, the remainder being essentially composed of the monoester in the 1 position.

9. D-Maltose monoesters according to Claim 8, characterized in that they are chosen from:
6'-O-octanoyl-D-maltose,
6'-O-hexadecanoyl-D-maltose,
6'-O-oleoyl-D-maltose,
6'-O-dodecanoyl-D-maltose, and
6'-O-tetradecanoyl-D-maltose.

10. Cosmetic, oral, pharmaceutical or foodstuff composition, characterized in that it comprises, in an appropriate vehicle, at least one D-maltose monoester predominantly in the 6' position of formula (II) according to Claims 8 and 9 or obtained according to any one of Claims 1 to 7.

11. Composition according to Claim 10, characterized in that the D-maltose monoester is present at a concentration of between 0.01 and 30% and preferably between 0.5 and 15% by weight with respect to the total weight of the composition.
